(19)

Europäisches Patentamt

European Patent Office

Office européen des brevets

(11)  **EP 4 622 557 B1**

(12)  **EUROPEAN PATENT SPECIFICATION**

(45) Date of publication and mention
of the grant of the patent:
**01.07.2026 Bulletin 2026/27**

(21) Application number: **23808738.1**

(22) Date of filing: **15.11.2023**

(51) International Patent Classification (IPC):
*A61B 8/08* (2006.01)

(52) Cooperative Patent Classification (CPC):
**A61B 8/5284; A61B 8/08; A61B 8/085;
A61B 8/5223**

(86) International application number:
**PCT/EP2023/081807**

(87) International publication number:
**WO 2024/110264 (30.05.2024 Gazette 2024/22)**

(54) **DIAPHRAGMATIC ULTRASONOGRAPHY IMAGE ALIGNMENT AND MOTION COMPENSATION
FOR CONTRALATERAL COMPARISON**

BILDAUSRICHTUNG UND BEWEGUNGSKOMPENSATION IN DER ZWERCHFELLSONOGRAPHIE
FÜR DEN KONTRALATERALEN VERGLEICH

ALIGNEMENT DE L'IMAGE DE L'ULTRASONOGRAPHIE DIAPHRAGMATIQUE ET
COMPENSATION DU MOUVEMENT POUR LA COMPARAISON CONTROLATÉRALE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **21.11.2022 US 202263426931 P**

(43) Date of publication of application:
**01.10.2025 Bulletin 2025/40**

(73) Proprietor: **Koninklijke Philips N.V.
5656 AE Eindhoven (NL)**

(72) Inventors:
• **WIEMKER, Rafael
5656 AG Eindhoven (NL)**
• **BUIZZA, Roberto
5656 AG Eindhoven (NL)**
• **HAARTSEN, Jaap Roger
5656 AG Eindhoven (NL)**
• **HENDRIKS, Cornelis Petrus
5656 AG Eindhoven (NL)**
• **KOEHLER, Thomas
5656AG Eindhoven (NL)**
• **WIEBERNEIT, Nataly
5656AG Eindhoven (NL)**
• **POLKEY, Michael
5656AG Eindhoven (NL)**

• **SABCZYNSKI, Jörg
5656AG Eindhoven (NL)**

(74) Representative: **Philips Intellectual Property &
Standards
High Tech Campus 34
5656 AE Eindhoven (NL)**

(56) References cited:
**US-A1- 2019 328 361**

• **MATAMIS DIMITRIOS ET AL: "Sonographic
evaluation of the diaphragm in critically ill
patients. Technique and clinical applications",
INTENSIVE CARE MEDICINE, SPRINGER
BERLIN HEIDELBERG, BERLIN/HEIDELBERG,
vol. 39, no. 5, 24 January 2013 (2013-01-24),
pages 801 - 810, XP037119915, ISSN: 0342-4642,
[retrieved on 20130124], DOI: 10.1007/
S00134-013-2823-1**
• **LE NEINDRE AYMERIC ET AL: "Thoracic
ultrasound: Potential new tool for
physiotherapists in respiratory management. A
narrative review", JOURNAL OF CRITICAL
CARE, GRUNE AND STRATTON, ORLANDO, FL,
US, vol. 31, no. 1, 26 October 2015 (2015-10-26),
pages 101 - 109, XP029351724, ISSN: 0883-9441,
DOI: 10.1016/J.JCRC.2015.10.014**

## Description

### CROSS-REFERENCE TO RELATED APPLICATIONS

This patent application claims the priority benefit under 35 U.S.C. § 119(e) of U.S. Provisional Application No. 63/426,931, filed on November 21, 2022.

The following relates generally to the respiratory therapy arts, mechanical ventilation arts, ventilator induced lung injury (VILI) prevention arts, ultrasound imaging arts, and related arts.

### BACKGROUND

Diaphragmatic ultrasonography (US) allows for quantification of diaphragm thickness, strain (rate) and excursion, and with this also the respiratory rate and duration of each contraction. Diaphragm thickness (expressed as thickening fraction) and strain reflect contractile activity and correlate well with diaphragmatic electrical activity and diaphragmatic pressure. Consequently, thickness and strain may be used as a surrogate for respiratory effort. Applications of diaphragmatic ultrasound include assessment of diaphragm function, atrophy detection, weaning prediction, and mechanical ventilation (MV) setting management. Other applications are asynchrony detection and proportional ventilation (non-invasive neurally adjusted ventilatory assist (NAVA)). The use of diaphragmatic ultrasound in mechanical ventilation is gaining attention and therefore, technical problems and use cases are currently being investigated.

A diaphragm thickening fraction (TFdi or TFDI) as measured by ultrasound (US) is carried out by an operator who looks at the patient and takes an ultrasound image at end inhalation and end exhalation. The diaphragm thickness fraction is typically determined by subtracting the end inhalation thickness $T_{ei}$ from the end exhalation thickness $T_{ee}$ and dividing the difference by the exhale thickness according to Equation 1:

$$TFdi = \frac{T_{ei} - T_{ee}}{T_{ee}} * 100\% \qquad (1)$$

The thickness of the diaphragm as varying over the breathing cycle (or more precisely the thickening during inspiration) is a surrogate for the patient's respiratory effort (see, e.g., Tuinman PR, Jonkman AH, Dres M, Shi ZH, Goligher EC, Goffi A, de Korte C, Demoule A, Heunks L. Respiratory muscle ultrasonography: methodology, basic and advanced principles and clinical applications in ICU and ED patients - a narrative review. Intensive Care Med. 2020 Apr;46(4):594-605. doi: 10.1007/s00134-019-05892-8. Epub 2020 Jan 14. PMID: 31938825; PMCID: PMC7103016.).

A diaphragm excursion (Edi) as well as the absolute thickening (Tdi) or fractional thickening (TFdi) as measured using a hand-held ultrasound probe are widely recognized parameters to assess diaphragm function, and to optimize ventilator support and weaning, as well as to assess and optimize the effect of artificial stimulation of the thoracic diaphragm using phrenic nerve stimulation (PNS) by implanted electric leads or non-invasively. The diaphragm thickening depends on diaphragmatic activity and reflects the diaphragmatic work of breathing (WoB) (i.e., the respiratory effort).

Diaphragmatic excursion as well as diaphragmatic thickness are both functions over the respiratory cycle, and independent clinical variables. Contra-lateral asymmetry (i.e., between left and right lung) may be an indication of medical complications (e.g., regional pulmonary tissue stiffness, regional diaphragmatic atrophy, phrenic nerve damage, etc.).

While diaphragmatic US with a handheld probe is widely practiced, the visual appraisal of the moving US image by the medical observer is confounded by certain complications. For example, excursion and thickening are two parameters which are difficult to appraise separately, because both change simultaneously, and thickness variation may be only a tenth of the motion amplitude. The diaphragm is constantly moving within the field of view (FOV), either being passively pushed by the mechanical ventilation, or actively driven by muscular activity. In another example, contralateral symmetry, or more general regional symmetries, are difficult to appraise visually with a single US probe, as typically the FOV of a hand-held US-probe covers only a part of either one of the left or right lung area and a corresponding portion of the extended diaphragm muscle. In another example, the effects of MV and phrenic nerve stimulation (PNS), respectively, as well as their gradual settings, are difficult to distinguish as both are supposed to cause a similar respiratory motion, however by different mechanisms. During the weaning process, the passive MV-caused part is desired to be reduced in favor of muscular motion, but the appearance differences may be subtle.

The following discloses certain improvements to overcome these problems and others.

US 2019/328361 A1 describes an ultrasound imaging system acquiring ultrasound image data from moving an ultrasound probe over a body of a person, and automatically dividing the ultrasound image data into segments of interest.

Matamis Dimitrios et al., "Sonographic evaluation of the diaphragm in critically ill patients. Technique and clinical applications", describes the technique and clinical applications of ultrasonography in the evaluation of diaphragmatic function in ICU patients.

### SUMMARY OF THE DISCLOSURE

The invention is set out in the appended set of claims.

In one aspect, there is provided a respiration monitoring device for monitoring a respiratory system of a patient, in which the respiratory system comprising a left lung, a right lung, and a thoracic diaphragm of the patient, according to claim 1.

In another aspect, there is provided a respiration monitoring method for monitoring a respiratory system of a patient, in which the respiratory system comprising a left lung, a right lung, and a thoracic diaphragm of the patient, according to claim 10.

One advantage resides in providing an intuitive and time-synchronized contralateral ultrasound-based visualization of left and right lungs and/or corresponding thoracic diaphragm muscle portions.

Another advantage resides in determining and visualizing contra-lateral asymmetry between matching organs (i.e., lungs) in a patient.

Another advantage resides in providing time-synchronized visualization of both the left and right lung (and/or corresponding diaphragm) portions using a user-selected synchronization signal, thereby providing intuitive visualization of information such as the extent to which different synchronization signals correlate with the dynamic motion of the lungs.

Another advantage resides in providing contra-lateral ultrasound visualization of a bilaterally symmetric organ or organ system such as the lungs or heart using only a single ultrasound probe with limited depth-of-penetration.

Another advantage resides in providing such a contra-lateral ultrasound visualization with a single ultrasound probe facilitating visual comparison of functioning of the left and right portions of the bilaterally symmetric organ or organ system.

Another advantage resides in distinguishing between effects on a patient from mechanical ventilation and phrenic nerve stimulation.

A given embodiment may provide none, one, two, more, or all of the foregoing advantages, and/or may provide other advantages as will become apparent to one of ordinary skill in the art upon reading and understanding the present disclosure.

## BRIEF DESCRIPTION OF THE DRAWINGS

The disclosure may take form in various components and arrangements of components, and in various steps and arrangements of steps. The drawings are only for purposes of illustrating the preferred embodiments and are not to be construed as limiting the disclosure.

FIGURE 1 diagrammatically shows a simplified representation of the left and right lungs and surrounding tissue.

FIGURE 2 diagrammatically shows an illustrative respiration monitoring device in accordance with the present disclosure.

FIGURES 3A and 3B show example flow charts of operations suitably performed by the device of FIGURE 1.

FIGURES 4 and 5 show different embodiments of image processing performed by the device of FIGURE 1.

FIGURE 6 diagrammatically shows a time-synchronized contralateral ultrasound-based visualization of left and right thoracic diaphragm muscle portions, along with the corresponding synchronization signal and a user dialog for selecting the synchronization signal.

## DETAILED DESCRIPTION

As used herein, the singular form of "a," "an," and "the" include plural references unless the context clearly dictates otherwise. As used herein, statements that two or more parts or components are "coupled," "connected," or "engaged" shall mean that the parts are joined, operate, or co-act together either directly or indirectly, i.e., through one or more intermediate parts or components, so long as a link occurs. Directional phrases used herein, such as, for example and without limitation, top, bottom, left, right, upper, lower, front, back, and derivatives thereof, relate to the orientation of the elements shown in the drawings and are not limiting upon the scope of the claimed invention unless expressly recited therein. The word "comprising" or "including" does not exclude the presence of elements or steps other than those described herein and/or listed in a claim. In a device comprised of several means, several of these means may be embodied by one and the same item of hardware.

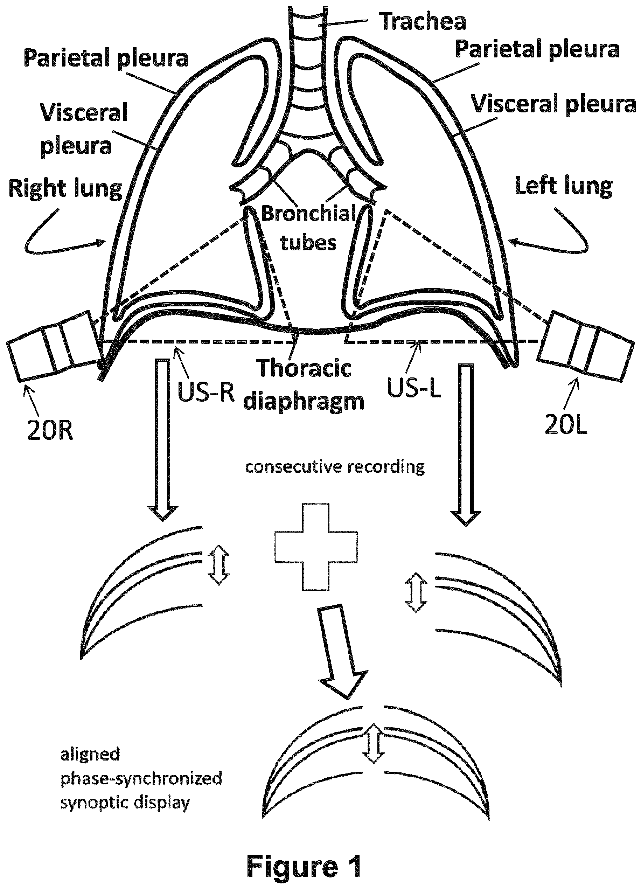

With brief initial reference to FIGURE 1, the upper portion thereof diagrammatically shows a simplified representation of the left and right lungs as delineated by the parietal and visceral pleura, the thoracic diaphragm (sometimes referred to herein as the diaphragm for brevity), and contextual anatomy including the trachea and upper portions of the left and right bronchial tubes. For further context, the visceral pleural is adhered to the lungs while the parietal pleura is adhered to the chest wall. As seen, the diaphragm extends underneath (i.e. is positioned anatomically inferior to) the lungs. The thoracic diaphragm is a muscular sheet that extends underneath both left and right lungs. In a healthy patient, during inhalation the diaphragm contracts to expand the lungs downward and thereby draw air into the lungs; during exhalation, the diaphragm relaxes, and the inflated lungs (at least partially) deflate to exhale the air. Hence, the contracting diaphragm thickens during inhalation and is typically at its greatest thickness at end-inspiration, and reduces in thickness during exhalation and is typically at its minimum thickness at end-expiration. By contrast, in the case of a patient who is unable to make any respiratory effort and is receiving mechanical ventilation, the thickening/thinning of the diaphragm over the breathing cycle is reduced or eliminated, although some diaphragm excursion and possibly limited diaphragm thickening/thinning may still occur in response to the mechanical ventilator-driven expansion and contraction of the lungs.

Also shown in the upper anatomical diagram of FIGURE 1 are a left-side-positioned ultrasound probe **20L** arranged to view the left lung and a corresponding portion of the thoracic diaphragm, and a corresponding right-side-positioned ultrasound probe **20R** arranged to view the right lung and a corresponding portion of the thoracic diaphragm. The left and right probe positions **20L** and **20R** are suitable positions of an external ultrasound probe used for monitoring the diaphragm motion. The illustrated left and right probe positions **20L** and **20R** are diagrammatic, and in practice various intercostal or subcostal positions may be employed for this purpose. As further illustrated, the left-side-positioned ultrasound probe **20L** emits an ultrasound fan-beam **US-L** that provides for imaging of a portion of the left lung and a corresponding portion of the diaphragm, and in contralateral fashion the right-side-positioned ultrasound probe **20R** emits an ultrasound fan-beam **US-R** that provides for imaging of a portion of the right lung and a corresponding portion of the diaphragm. (In three-dimensional ultrasound imaging, the fan-beams **US-L** and **US-R** may be cone-shaped beams). The fan-beams **US-L** and **US-R** intersect the diaphragm so as to be able to monitor movement and thickening of the diaphragm in real time. However, as shown typically neither of the fan-beams **US-L** and **US-R** penetrate deeply enough into the thorax to simultaneously image both the left and right lungs and corresponding portions of the diaphragm. Consequently, the operator can only observe one lung and corresponding diaphragm portion at any given time. This can be problematic, since observation of small differences between the dynamics of the left and right lungs (and/or corresponding left and right portions of the diaphragm) can be of substantial diagnostic value, for example facilitating diagnosis of an abnormality in one lung based on comparison with the other lung.

As diagrammatically shown in the lower portion of FIGURE 1 and as further described below with reference to illustrative embodiments described with reference to the various drawings, embodiments disclosed herein provide an improved visualization by way of combining presentation of a live ultrasound image as a function of time of a second portion of the respiratory system of the patient (for example, the left lung and/or left diaphragm portion of the patient) using the ultrasound probe in the left-position **20L.** If the ultrasound imaging device has two ultrasound probes, then one ultrasound probe could be placed in the left-position **20L** and the other ultrasound probe in the right-position **20R.** In this case, a presentation can be provided of a recorded ultrasound image as a function of time of both a first portion of the respiratory system acquired using the ultrasound probe located in the left-position **20L** and a second, contralateral portion of the respiratory system of the patient (in the instant example, the right lung and/or right diaphragm portion of the patient) acquired using the ultrasound probe located in the right-position **20R.** Advantageously, both images will be synchronized in time (assuming the ultrasound imaging device timestamps the imaging data acquired by the two ultrasound probes.)

However, many ultrasound systems only have a single ultrasound probe, and/or only provide for imaging using a single ultrasound probe at any given time. In this case, there is only one ultrasound probe, and hence imaging at any given time can be done with the probe in the left-position **20L** or the right-position **20R,** but not both simultaneously. In embodiments disclosed herein that accommodate such "single-probe" ultrasound imaging, a visualization is produced by way of combining presentation of a live ultrasound image as a function of time of a second portion of the respiratory system of the patient (for example, the left lung and/or left diaphragm portion of the patient) using the single ultrasound probe in the left-position **20L,** displayed simultaneously and in temporal alignment (by which it is meant respiratory phase alignment, not absolute time alignment; put another way, the temporal alignment is of respiratory cycling of the first ultrasound imaging data and the second ultrasound imaging data) with presentation of a recorded ultrasound image as a function of time of a second, contralateral portion of the respiratory system of the patient (in the instant example, the right lung and/or right diaphragm portion of the patient previously acquired with the single ultrasound probe in the right-position **20R**).

To enable this contralateral imaging using a single ultrasound probe, the recorded ultrasound image of the first, contralateral portion of the respiratory system is "pre-recorded," that is, recorded prior to acquisition of the live ultrasound image. In a variant embodiment, both contralateral portions of the respiratory system are pre-recorded, in which case no live ultrasound image is displayed. As further disclosed herein, the temporal alignment of the simultaneously displayed left and right ultrasound images may utilize various synchronization signals. A suitable synchronization signal is a one-dimensional signal (that is, value samples acquired as a function of time) cycling in correspondence with respiratory cycling of the respiratory system of the patient. By way of nonlimiting illustrative example, the synchronization signal may be a ventilator flow, pressure, volume, or work-of-breathing (WOB) signal recorded by a mechanical ventilator operating to mechanically ventilate the respiratory system of the patient, or a phrenic nerve stimulation (PNS) signal indicative of PNS administered to the patient, or a diaphragm thickness or excursion signal extracted from the first ultrasound imaging data and the second ultrasound imaging data of the respective first and second (i.e. contralateral) portions of the respiratory system.

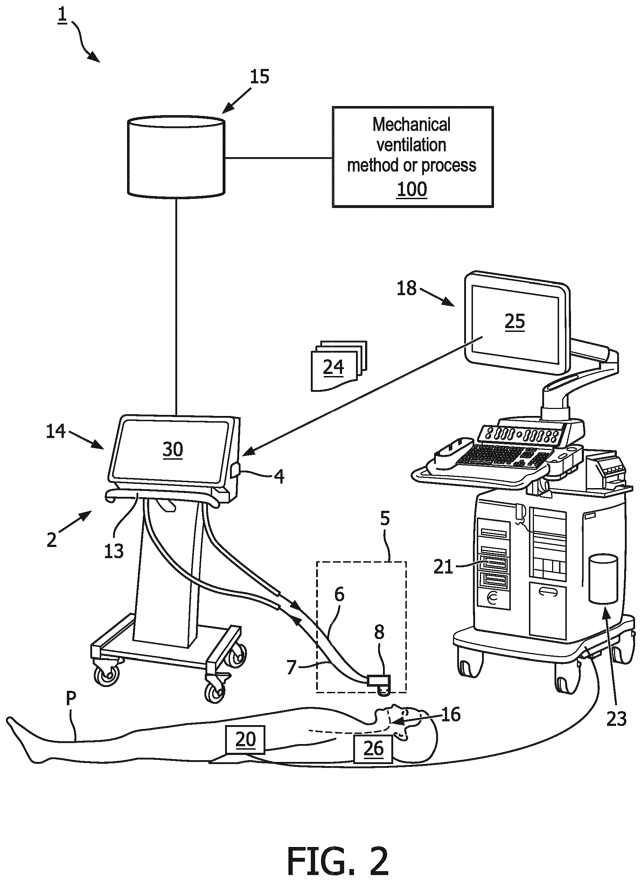

With reference to FIGURE 2, a mechanical ventilation or respiration monitoring device **1** is shown. A mechanical ventilator **2** is configured to provide ventilation therapy to an associated patient **P** is shown. As shown in FIGURE 2, the mechanical ventilator **2** includes an outlet **4** connectable with a patient breathing circuit **5** to delivery mechanical ventilation to the patient **P.** The patient breathing circuit **5** includes typical components for a mechanical ventilator, such as an inlet line **6,** an optional outlet line **7** (this may be omitted if the ventilator employs a single-limb patient circuit), a connector or port **8** for connecting with an endotracheal tube (ETT) **16,** and one or more breathing sensors (not shown), such as a gas flow meter, a pressure sensor, end-tidal carbon dioxide (etCO$_2$) sensor, and/or so forth. The mechanical ventilator **2** is designed to deliver air, an air-oxygen mixture, or other breathable gas (supply not shown) to the outlet **4** at a programmed pressure and/or flow rate to ventilate the patient via an ETT. The mechanical ventilator **2** also includes at least one electronic processor or controller **13** (e.g., an electronic processor or a microprocessor), a display device **14,** and a non-transitory computer readable medium **15** storing instructions executable by the electronic controller **13.**

FIGURE 2 diagrammatically illustrates the patient **P** intubated with an ETT **16** (the lower portion of which is inside the patient **P** and hence is shown in phantom). The connector or port **8** connects with the ETT **16** to operatively connect the mechanical ventilator **2** to deliver breathable air to the patient **P** via the ETT **16.** The mechanical ventilation provided by the mechanical ventilator **2** via the ETT **16** may be therapeutic for a wide range of conditions, such as various types of pulmonary conditions like emphysema or pneumonia, viral or bacterial infections impacting respiration such as a COVID-19 infection or severe influenza, cardiovascular conditions in which the patient **P** receives breathable gas enriched with oxygen, or so forth.

FIGURE 2 also shows a medical imaging device **18** (also referred to as an image acquisition device, imaging device, and so forth). As primarily described herein, the medical imaging device **18** comprises an ultrasound (US) medical imaging device **18.** The illustrative embodiments employ brightness mode (B-mode) ultrasound imaging to assess the diaphragm thickness metric. However, other types of ultrasound imaging or data are contemplated, such as motion mode (M-mode) data collected as a single ultrasound line over a time interval, or so forth.

In a more particular example, the medical imaging device **18** includes an ultrasound probe **20** that is configured to image the lungs and/or diaphragm of the patient **P.** The US probe **20** is positioned to acquire US imaging data (i.e., two-dimensional (2D) US images) **24** of the diaphragm and/or the lungs of the patient **P.** For example, the US probe **20** is configured to acquire imaging data of a diaphragm of the patient **P,** and more particularly 2D US imaging data related to a dimension (e.g., a position, a thickness, and so forth) of the diaphragm of a patient **P** during inspiration and expiration while the patient **P** undergoes mechanical ventilation therapy with the mechanical ventilator **2.** In another example, the US probe **20** is configured to acquire imaging data of the left lung and the right lung of the patient **P** during inspiration and expiration, and optionally during inspiration and expiration while the patient **P** undergoes mechanical ventilation therapy with the mechanical ventilator **2.**

In one example, the medical imaging device **18** includes an electronic processor **21** configured to control the ultrasound imaging device **18** to acquire the US images **24,** and also includes a non-transitory computer readable medium **23** storing instructions executable by the electronic processor **21.** The medical imaging device **18** can also include a display device **25.** In another example, the electronic processor **13** of the mechanical ventilator **2** controls the ultrasound imaging device **18** to receive the ultrasound imaging data **24** of the diaphragm of the patient **P** from the US probe **20.** The ultrasound probe **20** allows for continuous and automatic acquisition of the diaphragm thickness data (Tdi) or diaphragm excursion data from the acquired ultrasound imaging data **24.**

FIGURE 2 also diagrammatically indicates a phrenic nerve stimulation (PNS) device **26** implanted in the patient **P.** A PNS device **26** is typically an electrode implanted in the patient **P** and positioned to delivery PNS therapy to a left or right phrenic nerve (i.e., in a neck) of the patient **P.** The PNS device **26** is implanted at an earlier time during an earlier procedure prior to the mechanical ventilation therapy delivered to the patient **P.** The PNS device **26** is operatively in communication with the electronic controller **13** of the mechanical ventilator **2** and/or the electronic processor **21** of the ultrasound imaging device **18.**

The non-transitory computer readable medium **15** of the mechanical ventilator **2** and/or the non-transitory computer readable medium **23** of the US imaging device **18** stores instructions executable by the electronic controller **13** (and/or the electronic processor **21**) to perform a respiration monitoring method or process **100.** Although primarily described in terms of the electronic controller **13**/non-transitory computer readable medium **15** of the mechanical ventilator **2,** the method **100** can similarly be performed by the electronic processor **21**/non-transitory computer readable medium **23** of the US imaging device **18.**

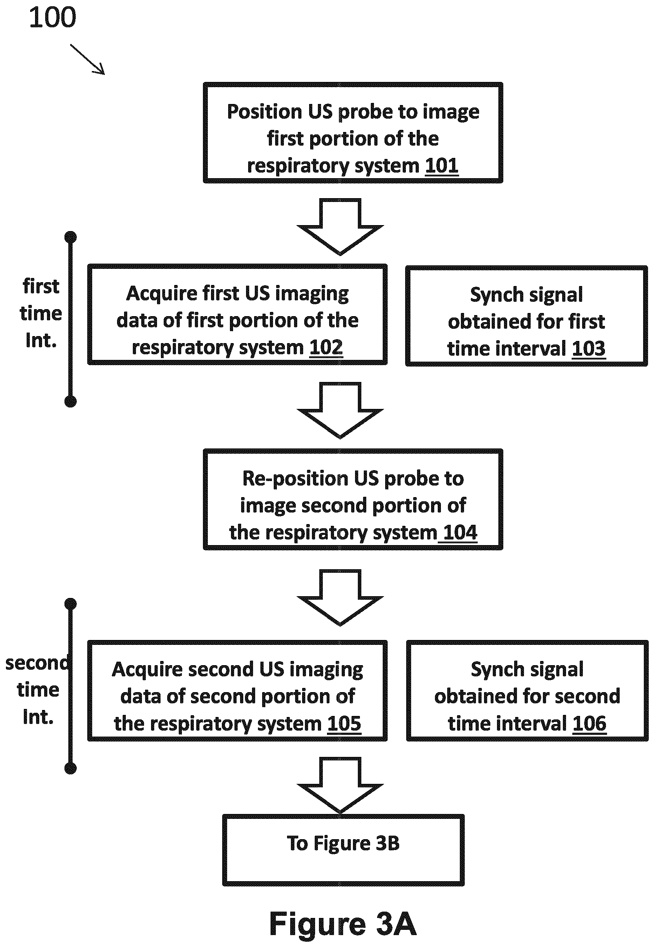

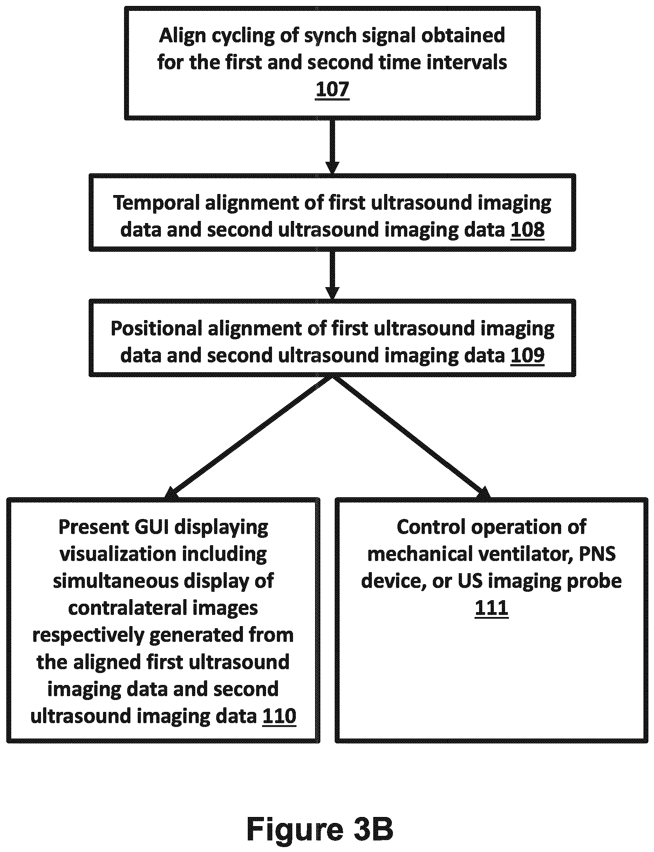

With reference to FIGURES 3A and 3B, and with continuing reference to FIGURE 2, an illustrative embodiment of the respiration monitoring method **100** is diagrammatically shown as a flowchart. Although described primarily in terms of the respiratory system of the patient **P** (e.g., a left lung, a right lung, and a thoracic diaphragm), the method **100** is also applicable to a cardiovascular system of the patient **P** (i.e., including at least a heart). The example of FIGURES 3A and 3B assumes that there is only the single US probe **20,** and/or that the ultrasound imaging device **18** can only acquire images using a single US probe at any given time. (As an example of the latter situation, the US imaging device **18** may be supplied with two or more US probes designed to provide different image characteristics or simply to provide a backup if one US probe fails; but only one of those two or more US probes can be connected with the US imaging device **18** and used to acquire US images at any given time).

As shown in FIGURE 3A, To begin the method **100,** at an operation **101,** the US probe **20** is positioned on the patient **P** to image a first portion of the respiratory system of the patient **P** (i.e., a right lung). At an operation **102,** first ultrasound imaging data **24** is acquired of the first portion of the respiratory system of the patient over a first time interval, with the ultrasound probe **20** and transmitted to, and received by, the electronic controller **13.** The first ultrasound imaging data **24** can be received by the electronic controller **13** as a function of time. In some embodiments, the US imaging data **24** is acquired while the patient **P** undergoes mechanical ventilation therapy with the mechanical ventilator **2.** The US imaging data **24** includes data related to a geometry (e.g., position, thickness, etc.) of the lungs and/or the diaphragm of the patient **P.** In a particular example, the **US** imaging data **24** includes a geometry (e.g., position, thickness, etc.) of both the right lung and the left lung of the patient **P** and include the diaphragm (in particular, movement data of the diaphragm during inhalation and exhalation by the patient **P**). To do so, the electronic controller **13** can control the ultrasound probe **20** to acquire the ultrasound imaging data **24** and to receive the ultrasound imaging data **24** of the diaphragm of the patient **P** from the ultrasound probe **20.** These images are not necessarily acquired while the patient **P** is on mechanical ventilation, but instead may be acquired (for example) prior to intubation of the patient.

At an operation **103,** a first synchronization signal for the first time interval is obtained. In some examples, the first synchronization signal can be a one-dimensional (1D) signal cycling in correspondence with respiratory cycling of the respiratory system of the patient **P.**

As further shown in FIGURE 3A, the operations **101-103** can be repeated for a second portion of the respiratory system of the patient that is contralateral to the first portion of the respiratory system of the patient (i.e., a left lung). At an operation **104,** the US probe **20** is positioned on the patient **P** to image the second portion of the respiratory system of the patient **P.** At an operation **105,** second ultrasound imaging data **24** is acquired of the second portion of the respiratory system of the patient over a second time interval, with the ultrasound probe **20** and transmitted to, and received by, the electronic controller **13.** The second ultrasound imaging data **24** can be received by the electronic controller **13** as a function of time. At an operation **106,** a first synchronization signal for the first time interval is obtained. In some examples, the first synchronization signal is can be a 1D signal cycling in correspondence with respiratory cycling of the respiratory system of the patient **P.**

In some embodiments, the first and/or second synchronization signals can be one or more of a ventilator flow, pressure, volume, or work-of-breathing signal recorded by the mechanical ventilator **2** operating over the first and second time intervals to mechanically ventilate the respiratory system of the patient **P;** a PNS signal indicative of PNS administered to the patient **P** by the PNS device **26** over the first and second time intervals; or a diaphragm thickness or excursion signal extracted from the first ultrasound imaging data **24** and the second ultrasound imaging data **24.** Although the operations **102** and **104** are described primarily as acquiring at least two regions of the first and second portions of the anatomy, ultrasound imaging data of two, three, four, or more regions can be acquired with the ultrasound probe **20.**

With continuing reference to FIGURES 2 and 3A, and referring now to FIGURE 3B, at an operation **107,** the electronic controller **13** is configured to align the cycling of the first and second synchronization signals. At an operation **108,** the first ultrasound imaging data **24** and the second ultrasound imaging data **24** can be aligned, for example, with a temporal alignment by aligning the cycling of the synchronization signal obtained over the first time interval and the cycling of the synchronization signal obtained over the second time interval.

In some embodiments, at an operation **109,** the electronic controller **13** is configured to align the first ultrasound imaging data **24** and the second ultrasound imaging data **24** further including positionally aligning the first ultrasound imaging data **24** and the second ultrasound imaging data **24** by aligning images of portions of the thoracic diaphragm represented in the respective first ultrasound imaging data **24** and second ultrasound imaging data **24.** To do so, the electronic controller **13** is configured to further includes performing image warping to remove a difference between diaphragm excursion over the respiratory cycling of the respiratory system of the patient **P** of the portions of the thoracic diaphragm represented in the respective first ultrasound imaging data **24** and second ultrasound imaging data **24.**

To align the cycling of the first and second synchronization signals, time-signal curves indicative of respiration data of the patient **P** over a time of US image acquisition are generated and displayed on the display device **14** of the mechanical ventilator **2** (and/or the display device **25** of the US imaging device **18**). To do so, individual one-dimensional (1D) time-signal curves are generated for the US imaging data **24** of a first (i.e., left) lung of the patient **P,** and a separate time-signal curve is generated for the US imaging data **24** of a second (i.e., right) lung of the patient **P.** The time-signal curve for the left lung and the time-signal curve for the right lung are synchronized based on corresponding image frames during acquisition of the ultrasound imaging data at a same time for the first lung and the second lung. For example, the image frame for the left lung acquired at t = 5 seconds is synchronized with the image frame for the right lung acquired at t = 5 seconds. The image frames of the time-signal curves for both lungs are similarly synchronized based on time, and the synchronized time-signal curve is displayed on the display device **14** of the mechanical ventilator **2** (and/or the display device **25** of the US imaging device **18**).

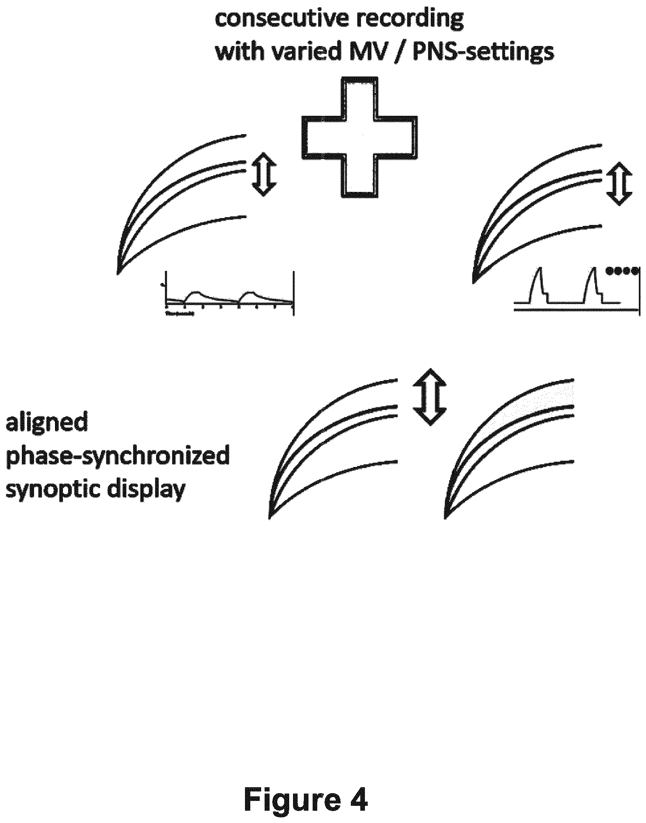

In a particular example, as illustrated in FIGURE 4, an aligned and synchronized time-signal curve after subsequent contralateral recordings of the lungs is generated. To do so, a clinician uses the US probe **20** to record one or more cycles on one patient side (i.e., the left lung), then moves the US probe **20** and observes on the opposite patient side (i.e., the right lung). During a subsequent observation, the US imaging data **24** is complemented in a synoptic display, showing two feeds (live & recorded) of two opposing patient sides, in a phase-synchronized fashion as shown in FIGURE 3. A live image is paired by the recorded contralateral image of the same respiratory phase selected based on MV treatment data. The clinician can toggle interactively between several available alignment signal curves (one out of the various available time-signal-curves). In addition, the paired contralateral phase-images are aligned vertically for optimized cross correlation in US-beam depth direction. This is achieved by storing with each image frame recorded during the US live feed (stream) the corresponding quantity of the various respiratory time-signal-curves. Then, during synoptic synchronized display, image frames are matched by their closest respiratory phase point. Optionally, for a given time frame of a first series, the best matching phase points A and B of a second series are identified, and an interpolation image between these closest frames is computed, to best match the frame of the first series.

In another example, all previously recorded respiratory cycles (or a live-feed) can be selected to be paired against each other in a synoptic display, i.e., in a phase-synchronized fashion. This allows a direct visual comparison of the effect of various settings on excursion and thickening. The user can toggle interactively between several available time-signal-curves to be used for image alignment. It is desirable that in two graphically juxta-posed US (echo) movies, the diaphragm appears at a similar vertical location in the opposing image frames (which may differ in the two FOVs, respectively). On the other hand, the relative diaphragm excursion in each frame may be desired to be retained. Therefore, a whole respiratory-cycle image-stack S1 (where each 2D echo image frame is considered as one slice of an image stack) is rigidly aligned against a second image-stack S2 by displacement along the x- and y-axes (with a fix z-axis). The optimal alignment shifts can be found by optimizing a e.g., mutual information, correlation, or another image agreement metric. In another embodiment, an object detection model is used (analytical or CNN-based such as YOLO) for explicit tracking of the diaphragm in both time series, and the mutual displacement is found by a regression of the 2D-coordinate curves.

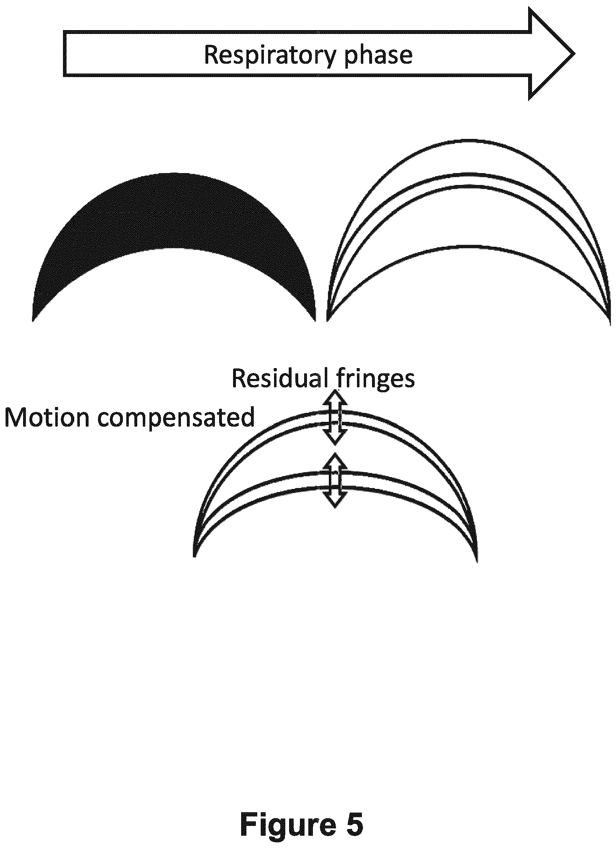

In some embodiments, the electronic controller **13** is configured to further align the first ultrasound imaging data **24** and the second ultrasound imaging data **24** further including performing motion correction to remove at least one uncorrelated motion component of at least one of the first ultrasound imaging data **24** and the second ultrasound imaging data **24.** The at least one uncorrelated motion component is not correlated with the cycling of the synchronization signal in correspondence with the respiration cycling of the lungs of the patient **P.** This is illustrated in FIGURE 5. The electronic controller **13** is configured to compensate for a spatially regularized (i.e., in some way uniform) shift along the beam depth, for depth shift patterns which are attributable (i.e., correlated) to the time signal curve from the mechanical ventilator **2.** Thus, the effect of the diaphragmatic excursion is removed, while diaphragmatic thickening is remaining as visible motion. The clinician can toggle interactively between several available time-signal-curves to be used for image alignment. This allows an appraisal of different causations for diaphragmatic motion, which, moreover, may differ regionally. Alternatively, the electronic controller **13** compensates for all local image deformation (i.e. not only vertical shifts, but also elastic local warps) which is attributable (i.e., correlated) to the time-signal-curve from the MV (or PNS). All image motions (excursion, thickening, etc.) which are moving in synch with the MV-time signal appear frozen, and residual flutter of the diaphragm lines remains visible in areas which are not governed by the selected phase signal curves. These residuals may reveal spontaneous (untriggered) breathing attempts of the ventilated patient **P.** For this option, it is desirable to compensate for the diaphragm excursion, while preserving variations in thickness. Therefore, a fully elastic image registration cannot be used. Rather, the medial line of the diaphragm is automatically detected and delineated in each image frame (using analytical or CNN-based semantic segmentation), and the 2D image is deformed (i.e., warped) such as to map the medial line onto a fix straight line or onto the line of a reference image.

For this embodiment, it is desired to filter out any motion in the image which is governed (correlated) by one of the available respiratory phase curves from MV, PNS, etc. Visual appraisal of the residual image movies can thus tell which of the devices appears to be most influential. The decorrelation is be achieved by Independent Component Analysis (ICP), Non-Negative Matrix Factorization (NNMF) (see, e.g., Rabbi, Mohammad & Pizzolato, Claudio & Lloyd, David & Carty, Christopher & Devaprakash, Daniel & Diamond, Laura, Non-negative matrix factorisation is the most appropriate method for extraction of muscle synergies in walking and running. Nature Scientific Reports, 2020.), or other algebraic techniques, which decompose the stack of image frames into a function of the phase vector. The phase vector is then set to uniformity, and the image frame are re-composed. In another embodiment, spatio-temporal Generative Models (GAN) or Variational Autoencoders (VAE) are trained to reproduce the observed image frames from one of the available respiratory time-signal-curves. Then the respiratory signal curve is squashed (flattened to uniformity), and virtual image frames are inferenced by the trained model. (This is not an off-site multi-case training, but rather an on-site on-the-fly case-specific training.)

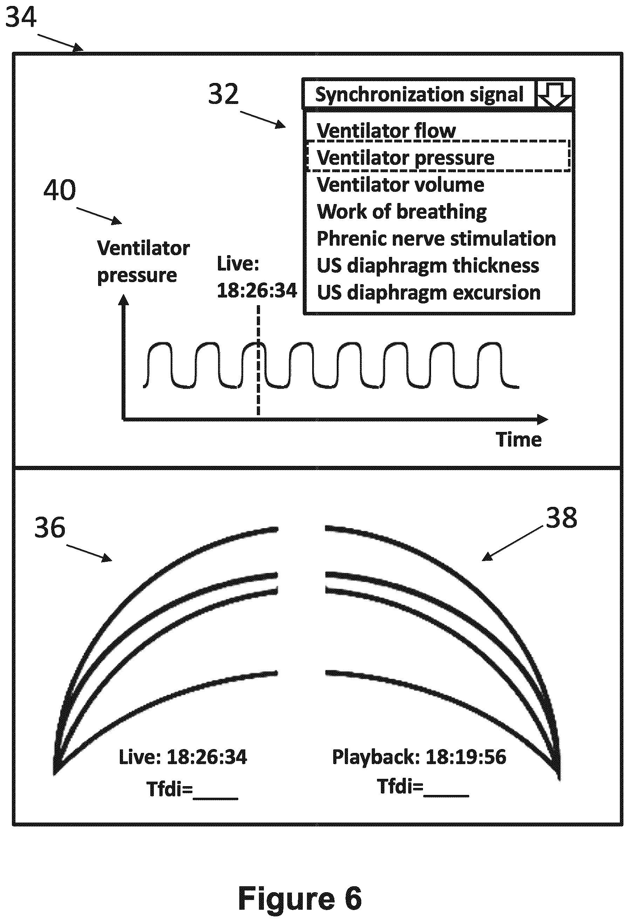

In some embodiments, the electronic controller **13** is configured to obtain a plurality of different signals each of which is a one-dimensional signal cycling in correspondence with respiration cycling of the respiratory system of the patient **P,** and select the synchronization signal from the obtained plurality of different signals. To do so, in some embodiments, a graphical user interface (GUI) **30** is presented on the display device **14** of the mechanical ventilator **2,** and a user dialog **32** (see FIGURE 6, described in more detail below) is presented on the GUI **30** by which the synchronization signal can be selected by a clinician. The selection of the synchronization signal is received, via the user dialog **32.** In another embodiment, the electronic controller **13** can automatically select the synchronization signal from the plurality of synchronization signals. In some examples, the alignment of the cycling of the synchronization signal and the temporal alignment of the first ultrasound imaging data **24** and the second ultrasound imaging data **24** are updated each time a different selection of the synchronization signal is received via the user dialog **32.**

At an operation **110,** a visualization **34** of at least a portion of the respiratory system of the patient **P.** As further shown in FIGURE 6, the visualization **34** includes a simultaneous display of an image **36** of the first portion of the respiratory system of the patient and an image **38** of the second portion of the respiratory system of the patient respectively generated from the aligned first ultrasound imaging data **24** and second ultrasound imaging data **24.** The user dialog **32** is shown in an upper right portions of the visualization **34** as shown in FIGURE 6. In some embodiments (as shown in the lower portion of FIGURE 6), the image **36** of the first portion of the respiratory system and the image **38** of the second portion of the respiratory system are cine images respectively generated from the temporally aligned first ultrasound imaging data **24** and second ultrasound imaging data **24.**

In some embodiments, the electronic controller **13** is configured to determine a first value of at least one thoracic diaphragm parameter for the first portion of the respiratory system of the patient from the first ultrasound imaging data **24,** and determine a second value of the at least one thoracic diaphragm parameter for the second portion of the respiratory system of the patient from the second ultrasound imaging data **24.** The visualization **32** further includes display of representations of the first and second values of the at least one thoracic diaphragm parameter, (as shown in the lower portion of FIGURE 6). As shown in FIGURE 4, the thoracic diaphragm parameter(s) can include one or more of (i) a measure of thoracic diaphragm thickness and/or (ii) a measure of thoracic diaphragm excursion. To do so, the electronic controller **13** is configured to receive treatment data associated with the diaphragm and/or the lungs of the patient **P.** In one example, the treatment data comprises one or more of (i) mechanical ventilation (MV) data of the patient **P** while the patient undergoes MV therapy with the mechanical ventilator **2;** and/or (ii) PNS data of the patient **P** while the patient **P** undergoes PNS therapy with the PNS device **26.** The MV data can include, for example, a respiratory pressure of the lungs of the patient **P,** a flow of air into the lungs of the patient **P,** a lung volume of the patient **P,** a works-of-breathing (WoB) time-curve, and so forth. The PNS data can include, for example, an electrical stimulation curve of electrical stimulation supplied by the PNS device **26.**

In some embodiments, as show in the upper portion of FIGURE 6, the visualization **34** further includes a trendline **40** of the synchronization signal obtained over one or both of the first and second time intervals together with the displayed visualization. The trendline **40** can, in some examples, be a color-coded representation of a magnitude of a correlation between time-signal curves over a time period of a respiratory phase of the patient can be displayed on the display device **14.** To do so, the first and second US imaging data **24** (or a recorded moving US image series) is displayed with a spatially resolved color overlay, indicating the magnitude of correlation of the local optical flow with the respiratory phase. A transparent overlay can be used for vanishing correlation (leaving the standard gray-value image), color opacity proportional to local correlation or covariance, color hue signifying the correlation source (MV, PNS, etc.). The clinician may toggle interactively between several available time-signal-curves to be used for image alignment. This allows an appraisal of the different correlation regions, respectively. This is achieved by computing an optical flow for each frame pixel, to extract the flow curve across the frame stack (phase-curve for each pixel), and to cross-correlate this curve against the available time-signal-curves.

In some embodiments, the first time interval is earlier than the second time interval, and the alignment of the cycling of the synchronization signal, the temporal alignment of the first ultrasound imaging data and the second ultrasound imaging data, and the displaying of the visualization **32** are performed in real time during the second time interval. In other embodiments, the alignment of the cycling of the synchronization signal, the temporal alignment of the first ultrasound imaging data and the second ultrasound imaging data, and the displaying of the visualization are performed entirely after completion of both the first time interval and the second time interval.

At an operation **111,** operation of a device can be controlled based on the temporally aligned first ultrasound imaging data **24** and second ultrasound imaging data **24.** For example, the mechanical ventilator **2** is controlled to adjust one or more parameters of the mechanical ventilation therapy delivered to the patient **P** based on the temporally aligned first ultrasound imaging data **24** and second ultrasound imaging data **24**. In another example, the US imaging probe **20** is controlled to acquire additional imaging data of the diaphragm and lungs of the patient **P** based on the temporally aligned first ultrasound imaging data **24** and second ultrasound imaging data **24**. In a further example, the PNS device **26** is controlled to adjust one or more parameters of the PNS therapy delivered to the patient **P** based on the temporally aligned first ultrasound imaging data **24** and second ultrasound imaging data **24**.

The embodiment of FIGURES 3A and 3B is suitably used when only one US probe **20** is available and/or when only one US probe can be used for imaging at any given time. In another contemplated embodiment (not shown), if two US probes are available then imaging can be simultaneously performed with one US probe positioned at the left-position **20L** (see FIGURE 1) and the other US probe positioned at the right-position **20R**. In this case, the acquired left and right cine images are inherently time-synchronized, and so the acquisition operations **102** and **105** of FIGURE 3A can be performed simultaneously (so that the first and second time intervals are the same) and there is no need for the operations **103, 104, 105, 107,** and **108**. The optional positional alignment **109** is still done in this embodiment, and the visualization **110** is provided as already described.

The disclosure has been described with reference to the preferred embodiments. Modifications and alterations may occur to others upon reading and understanding the preceding detailed description. It is intended that the exemplary embodiment be construed as including all such modifications and alterations insofar as they come within the scope of the appended claims.

## Claims

1. A respiration monitoring device (1) for monitoring a respiratory system of a patient, the respiratory system comprising a left lung, a right lung, and a thoracic diaphragm of the patient, the respiration monitoring device comprising an electronic controller (13) configured to:

   receive first ultrasound imaging data (24) as a function of time acquired of a first portion of the respiratory system of the patient over a first time interval;
   receive second ultrasound imaging data (24) as a function of time acquired of a second portion of the respiratory system of the patient over a second time interval wherein the second portion of the respiratory system of the patient is contralateral to the first portion of the respiratory system of the patient;
   obtain a synchronization signal for the first time interval and the second time interval wherein the synchronization signal is a one-dimensional signal cycling in correspondence with the respiratory cycling of the respiratory system of the patient;
   align the cycling of the synchronization signal obtained over the first time interval and the cycling of the synchronization signal obtained over the second time interval;
   temporally align respiratory cycling of the first ultrasound imaging data and the second ultrasound imaging data; and
   present a graphical user interface, GUI, (30) displaying a visualization (34) including simultaneous display of an image (36) of the first portion of the respiratory system of the patient and an image (38) of the second portion of the respiratory system of the patient respectively generated from the first ultrasound imaging data and second ultrasound imaging data;

wherein:

   the second time interval is different from the first time interval;
   the simultaneous display of the image of the first portion of the respiratory system of the patient and the image of the second portion of the respiratory system of the patient are respectively generated from the temporally aligned first ultrasound imaging data and second ultrasound imaging data; and
   the aligned cycling of the synchronization signal is used to temporally align the respiratory cycling of the first ultrasound imaging data and the second ultrasound imaging data; and

**characterized in that** the synchronization signal is:

   a phrenic nerve stimulation (PNS) signal indicative of PNS administered to the patient over the first and second time intervals; or
   a diaphragm thickness or excursion signal extracted from the first ultrasound imaging data and the second ultrasound imaging data.

2. The respiration monitoring device of claim 1, wherein one of:

   the first time interval is earlier than the second time interval, and the alignment of the cycling of the synchronization signal, the temporal alignment of the first ultrasound imaging data and the second ultrasound imaging data, and the displaying of the visualization are performed in real time during the second time interval; and
   the alignment of the cycling of the synchronization signal, the temporal alignment of the first ultrasound imaging data and the second ultrasound imaging data, and the displaying of the visualization are performed entirely after completion of both the first time interval and the second time interval.

3. The respiration monitoring device of claim 1, wherein, in the displayed visualization:

the image of the first portion of the respiratory system and the image of the second portion of the respiratory system are cine images respectively generated from the first ultrasound imaging data and second ultrasound imaging data; and

the cine image of the second portion of the respiratory system of the patient is positioned contralaterally to the cine image of the first portion of the respiratory system of the patient in correspondence to the contralateral arrangement of the second portion of the respiratory system of the patient to the first portion of the respiratory system of the patient.

4. The respiration monitoring device of claim 1, wherein the electronic controller is further configured to:

determine a first value of at least one thoracic diaphragm parameter for the first portion of the respiratory system of the patient from the first ultrasound imaging data; and

determine a second value of the at least one thoracic diaphragm parameter for the second portion of the respiratory system of the patient from the second ultrasound imaging data;

wherein the visualization further includes display of representations of the first and second values of the at least one thoracic diaphragm parameter.

5. The respiration monitoring device of claim 4, wherein the at least one thoracic diaphragm parameter includes at least one of (i) a measure of thoracic diaphragm thickness and/or (ii) a measure of thoracic diaphragm excursion.

6. The respiration monitoring device of claim 1, wherein the electronic controller is configured to:
positionally align the first ultrasound imaging data and the second ultrasound imaging data by aligning images of portions of the thoracic diaphragm represented in the respective first ultrasound imaging data and second ultrasound imaging data.

7. The respiration monitoring device of claim 6, wherein the positional aligning further includes performing image warping to remove a difference between diaphragm excursion over the respiratory cycling of the respiratory system of the patient of the portions of the thoracic diaphragm represented in the respective first ultrasound imaging data and second ultrasound imaging data.

8. The respiration monitoring device of claim 1, wherein the electronic controller is configured to:
perform motion correction to remove at least one motion component of at least one of the first ultrasound imaging data and the second ultrasound imaging data, wherein the at least one removed motion component is not correlated with respiration cycling of the lungs of the patient.

9. The respiration monitoring device of claim 1, further including at least one of:

an ultrasound imaging device having an ultrasound imaging probe arrangeable respective to the first lung and/or first portion of the thoracic diaphragm of the patient to acquire the first ultrasound imaging data and arrangeable respective to the second lung and/or second portion of the thoracic diaphragm of the patient to acquire the second ultrasound imaging data, wherein the ultrasound imaging probe is controlled to acquire additional imaging data of the diaphragm of the patient based on the temporally aligned first ultrasound imaging data and second ultrasound imaging data;

a mechanical ventilator configured to deliver mechanical ventilation therapy to the patient, wherein receiving the imaging data of a dimension of the lungs of occurs during inspiration and expiration while the patient undergoes mechanical ventilation therapy with the mechanical ventilator; wherein the mechanical ventilator is controlled to adjust one or more parameters of the mechanical ventilation therapy delivered to the patient based on the temporally aligned first ultrasound imaging data and second ultrasound imaging data; and

a phrenic nerve stimulation (PNS) device configured to provide PNS treatment to the patient, wherein the PNS device is controlled to adjust one or more parameters of the PNS therapy delivered to the patient based on the temporally aligned first ultrasound imaging data and second ultrasound imaging data.

10. A respiration monitoring method (100) for monitoring a respiratory system of a patient, the respiratory system comprising a left lung, a right lung, and a thoracic diaphragm of the patient, the respiration monitoring method comprising:

receiving first ultrasound imaging data (102) as a function of time acquired of a first portion of the respiratory system of the patient over a first time interval;

receiving second ultrasound imaging data (105) as a function of time acquired of a second portion of the respiratory system of the patient over a second time interval wherein the second portion of the respiratory system of the patient is contralateral to the first portion of the respiratory system of the patient;

obtaining a synchronization signal (103) for the first time interval and the second time interval wherein the synchronization signal is a one-dimensional signal cycling in correspondence with the respiratory cycling of the respiratory system of the patient;

aligning (107) the cycling of the synchronization signal obtained over the first time interval and the cycling of the synchronization signal obtained over the second time interval;

temporally aligning (108) respiratory cycling of the first ultrasound imaging data and the second ultrasound imaging data; and

presenting a graphical user interface (GUI) displaying (110) a visualization including simultaneous display of an image of the first portion of the respiratory system of the patient and an image of the second portion of the respiratory system of the patient respectively generated from the first ultrasound imaging data and second ultrasound imaging data;

wherein:

the second time interval is different from the first time interval;

the simultaneous display of the image of the first portion of the respiratory system of the patient and the image of the second portion of the respiratory system of the patient are respectively generated from the temporally aligned first ultrasound imaging data and second ultrasound imaging data; and

the aligned cycling of the synchronization signal is used to temporally align the respiratory cycling of the first ultrasound imaging data and the second ultrasound imaging data; and

**characterized in that** the synchronization signal is:

a phrenic nerve stimulation (PNS) signal indicative of PNS administered to the patient over the first and second time intervals; or

a diaphragm thickness or excursion signal extracted from the first ultrasound imaging data and the second ultrasound imaging data.

**Patentansprüche**

1. Atmungsüberwachungsvorrichtung (1) zur Überwachung eines Atmungssystems eines Patienten, wobei das Atmungssystem eine linke Lunge, eine rechte Lunge und ein Zwerchfell des Patienten umfasst, wobei die Atmungsüberwachungsvorrichtung eine elektronische Steuereinheit (13) umfasst, die konfiguriert ist zum:

Empfangen von ersten Ultraschallbildgebungsdaten (24) als Funktion von Zeit, die von einem ersten Abschnitt des Atmungssystems des Patienten über ein erstes Zeitintervall erfasst wurden;

Empfangen von zweiten Ultraschallbildgebungsdaten (24) als Funktion von Zeit, die von einem zweiten Abschnitt des Atmungssystems des Patienten über ein zweites Zeitintervall erfasst wurden, wobei der zweite Abschnitt des Atmungssystems des Patienten kontralateral zum ersten Abschnitt des Atmungssystems des Patienten ist;

Erhalten eines Synchronisationssignals für das erste Zeitintervall und das zweite Zeitintervall, wobei das Synchronisationssignal ein eindimensionales Signal ist, das sich entsprechend der Atmungswiederholung des Atmungssystems des Patienten wiederholt;

Ausrichten der Wiederholungen des über das erste Zeitintervall erhaltenen Synchronisationssignals und der Wiederholungen des über das zweite Zeitintervall erhaltenen Synchronisationssignals;

zeitliches Ausrichten der Atmungswiederholungen der ersten Ultraschallbildgebungsdaten und der zweiten Ultraschallbildgebungsdaten; und

Darstellen einer grafischen Benutzeroberfläche, GUI, (30) die eine Visualisierung (34) anzeigt, die gleichzeitiges Anzeigen eines Bildes (36) des ersten Abschnitts des Atmungssystems des Patienten und eines Bildes (38) des zweiten Abschnitts des Atmungssystems des Patienten einschließt, die jeweils aus den ersten Ultraschallbildgebungsdaten und den zweiten Ultraschallbildgebungsdaten erzeugt wurden;

wobei:

das zweite Zeitintervall vom ersten Zeitintervall unterschiedlich ist;

die gleichzeitige Anzeige des Bildes des ersten Abschnitts des Atmungssystems des Patienten und des Bildes des zweiten Abschnitts des Atmungssystems des Patienten jeweils aus den zeitlich ausgerichteten ersten Ultraschallbildgebungsdaten und zweiten Ultraschallbildgebungsdaten erzeugt wird; und

die ausgerichteten Wiederholungen des Synchronisationssignals verwendet werden, um die Atmungswiederholungen der ersten Ultraschallbildgebungsdaten und der zweiten Ultraschallbildgebungsdaten zeitlich auszurichten; und

**gekennzeichnet dadurch, dass** das Synchronisationssignal Folgendes ist:

ein Signal der Zwerchfellnervenstimulation (PNS), das eine dem Patienten im ersten und zweiten Zeitintervall verabreichte PNS angibt; oder
ein aus den ersten Ultraschallbildgebungsdaten und zweiten Ultraschallbildgebungsdaten extrahiertes Signal von Zwerchfelldicke oder -auslenkung.

2. Atmungsüberwachungsvorrichtung nach Anspruch 1, wobei eines:

des ersten Zeitintervalls früher ist als das zweite Zeitintervall und die Ausrichtung der Wiederholungen des Synchronisationssignals, die zeitliche Ausrichtung der ersten Ultraschallbildgebungsdaten und zweiten Ultraschallbildgebungsdaten und das Anzeigen der Visualisierung in Echtzeit während des zweiten Zeitintervalls durchgeführt werden; und
die Ausrichtung der Wiederholungen des Synchronisationssignals, die zeitliche Ausrichtung der ersten Ultraschallbildgebungsdaten und zweiten Ultraschallbildgebungsdaten und das Anzeigen der Visualisierung vollständig nach Beendigung sowohl des ersten Zeitintervalls als auch des zweiten Zeitintervalls durchgeführt werden.

3. Atmungsüberwachungsvorrichtung nach Anspruch 1, wobei in der angezeigten Visualisierung:

das Bild des ersten Abschnitts des Atmungssystems und das Bild des zweiten Abschnitts des Atmungssystems Cine-Bilder sind, die jeweils aus den ersten Ultraschallbildgebungsdaten und zweiten Ultraschallbildgebungsdaten generiert wurden; und
das Cine-Bild des zweiten Abschnitts des Atmungssystems des Patienten kontralateral zum Cine-Bild des ersten Abschnitts des Atmungssystems des Patienten in Entsprechung mit der kontralateralen Anordnung des zweiten Abschnitts des Atmungssystems des Patienten zum ersten Abschnitt des Atmungssystems des Patienten positioniert ist.

4. Atmungsüberwachungsvorrichtung Anspruch 1, wobei die elektronische Steuereinheit weiter konfiguriert ist zum:

Bestimmen eines ersten Werts von mindestens einem Parameter des Zwerchfells für den ersten Abschnitt des Atmungssystems des Patienten aus den ersten Ultraschallbildgebungsdaten; und
Bestimmen eines zweiten Werts des mindestens einen Parameters des Zwerchfells für den zweiten Abschnitt des Atmungssystems des Patienten aus den zweiten Ultraschallbildgebungsdaten;
wobei die Visualisierung weiter Anzeigen von Darstellungen des ersten Werts und des zweiten Wertes des mindestens einen Parameters des Zwerchfells einschließt.

5. Atemüberwachungsvorrichtung nach Anspruch 4, wobei der mindestens eine Parameter des Zwerchfells mindestens eines einschließt von (i) einem Maß von Dicke des Zwerchfells und/oder (ii) einem Maß von Auslenkung des Zwerchfells.

6. Atmungsüberwachungsvorrichtung nach Anspruch 1, wobei die elektronische Steuereinheit konfiguriert ist zum:
positionellen Ausrichten der ersten Ultraschallbildgebungsdaten und der zweiten Ultraschallbildgebungsdaten durch Ausrichten von Bildern von Abschnitten des Zwerchfells, die in den jeweiligen ersten Ultraschallbildgebungsdaten und zweiten Ultraschallbildgebungsdaten dargestellt sind.

7. Atmungsüberwachungsvorrichtung nach Anspruch 6, wobei das positionelle Ausrichten weiter Durchführen von Bildverzerrung einschließt, um einen Unterschied zwischen Zwerchfellauslenkung über die Atmungswiederholung des Atmungssystems des Patienten der Abschnitte des Zwerchfells, die jeweils in den ersten Ultraschallbildgebungsdaten und zweiten Ultraschallbildgebungsdaten dargestellt sind, zu entfernen.

8. Atmungsüberwachungsvorrichtung nach Anspruch 1, wobei die elektronische Steuereinheit konfiguriert ist zum:
Durchführen von Bewegungskorrektur, um mindestens eine Bewegungskomponente mindestens eines der ersten Ultraschallbildgebungsdaten und der zweiten Ultraschallbildgebungsdaten zu entfernen, wobei die mindestens eine entfernte Bewegungskomponente nicht mit Atmungswiederholung der Lunge des Patienten korreliert ist.

9. Atmungsüberwachungsvorrichtung nach Anspruch 1, die weiter mindestens eines von Folgendem einschließt:

eine Ultraschallbildgebungsvorrichtung, die eine Ultraschallbildgebungssonde aufweist, die in Bezug auf die erste Lunge und/oder den ersten Abschnitt des Zwerchfells des Patienten angeordnet werden kann, um die ersten Ultraschallbildgebungsdaten zu erfassen, und in Bezug auf die zweite Lunge und/oder den zweiten Abschnitt des Zwerchfells des Patienten angeordnet werden kann, um die zweiten Ultraschallbildgebungsdaten zu erfassen, wobei die Ultraschallbildgebungssonde so gesteuert wird, dass sie zusätzliche Bildgebungsdaten des Zwerchfells des Patienten basierend auf den zeitlich ausgerichteten ersten und zweiten Ultraschallbildgebungsdaten erfasst;
ein mechanisches Beatmungsgerät, das so konfiguriert ist, dass es Abgabe von mechanischer Beatmungstherapie an den Patienten durchführt, wobei Empfangen der Bildgebungsdaten einer Lungendimension während Inspiration und Ausatmung erfolgt, während der Patient mit dem mechanischen Beatmungsgerät mechanischer Beatmungstherapie unterzogen wird; wobei das mechanische Beatmungsgerät so gesteuert wird, dass es einen oder mehrere Parameter der an den Patienten abgegebenen mechanischen Beatmungstherapie basierend auf den zeitlich ausgerichteten ersten und zweiten Ultraschallbildgebungsdaten anpasst; und eine Vorrichtung zur Zwerchfellnervenstimulation (PNS), die so konfiguriert ist, dass sie dem Patienten PNS-Behandlung bereitstellt, wobei die PNS-Vorrichtung so gesteuert wird, dass sie einen oder mehrere Parameter der an den Patienten abgegebenen PNS-Therapie basierend auf den zeitlich ausgerichteten ersten Ultraschallbildgebungsdaten und zweiten Ultraschallbildgebungsdaten anpasst.

10. Atmungsüberwachungsverfahren (100) zur Überwachung eines Atmungssystems eines Patienten, wobei das Atmungssystem eine linke Lunge, eine rechte Lunge und ein Zwerchfell des Patienten umfasst, wobei das Atmungsüberwachungsverfahren Folgendes umfasst:

Empfangen von ersten Ultraschallbildgebungsdaten (102) als Funktion von Zeit, die von einem ersten Abschnitt des Atmungssystems des Patienten über ein erstes Zeitintervall erfasst wurden;
Empfangen von zweiten Ultraschallbildgebungsdaten (105) als Funktion von Zeit, die von einem zweiten Abschnitt des Atmungssystems des Patienten über ein zweites Zeitintervall erfasst wurden, wobei der zweite Abschnitt des Atmungssystems des Patienten kontralateral zum ersten Abschnitt des Atmungssystems des Patienten ist;
Erhalten eines Synchronisationssignals (103) für das erste Zeitintervall und das zweite Zeitintervall, wobei das Synchronisationssignal ein eindimensionales Signal ist, das sich entsprechend der Atmungswiederholung des Atmungssystems des Patienten wiederholt;
Ausrichten (107) der Wiederholungen des über das erste Zeitintervall erhaltenen Synchronisationssignals und der Wiederholungen des über das zweite Zeitintervall erhaltenen Synchronisationssignals;
zeitliches Ausrichten (108) der Atmungswiederholungen der ersten Ultraschallbildgebungsdaten und der zweiten Ultraschallbildgebungsdaten; und
Darstellen einer grafischen Benutzeroberfläche (GUI), die eine Visualisierung anzeigt (110), die gleichzeitiges Anzeigen eines Bildes des ersten Abschnitts des Atmungssystems des Patienten und eines Bildes des zweiten Abschnitts des Atmungssystems des Patienten einschließt, die jeweils aus den ersten Ultraschallbildgebungsdaten und den zweiten Ultraschallbildgebungsdaten erzeugt wurden;
wobei:

das zweite Zeitintervall vom ersten Zeitintervall unterschiedlich ist;
die gleichzeitige Anzeige des Bildes des ersten Abschnitts des Atmungssystems des Patienten und des Bildes des zweiten Abschnitts des Atmungssystems des Patienten jeweils aus den zeitlich ausgerichteten ersten Ultraschallbildgebungsdaten und zweiten Ultraschallbildgebungsdaten erzeugt wird; und
die ausgerichteten Wiederholungen des Synchronisationssignals verwendet werden, um die Atmungswiederholungen der ersten Ultraschallbildgebungsdaten und der zweiten Ultraschallbildgebungsdaten zeitlich auszurichten; und

**gekennzeichnet dadurch, dass** das Synchronisationssignal Folgendes ist:

ein Signal der Zwerchfellnervenstimulation (PNS), das eine dem Patienten im ersten und zweiten Zeitintervall verabreichte PNS angibt; oder
ein aus den ersten Ultraschallbildgebungsdaten und zweiten Ultraschallbildgebungsdaten extrahiertes Signal von Zwerchfelldicke oder -auslenkung.

## Revendications

1. Dispositif de surveillance de la respiration (1) pour surveiller le système respiratoire d'un patient, le système respiratoire comprenant le poumon gauche, le poumon droit et le diaphragme thoracique du patient, le dispositif de surveillance de la respiration comprenant un dispositif de commande électronique (13) configuré pour :

recevoir des premières données d'imagerie ultrasonore (24) en fonction du temps acquises d'une première partie du système respiratoire du patient sur un premier intervalle de temps ;

recevoir des secondes données d'imagerie ultrasonore (24) en fonction du temps acquises d'une seconde partie du système respiratoire du patient sur un second intervalle de temps, dans lequel la seconde partie du système respiratoire du patient est controlatérale à la première partie du système respiratoire du patient ;

obtenir un signal de synchronisation pour le premier intervalle de temps et le second intervalle de temps, dans lequel le signal de synchronisation est un cycle de signal unidimensionnel en correspondance avec le cycle respiratoire du système respiratoire du patient ;

aligner le cycle du signal de synchronisation obtenu sur le premier intervalle de temps et le cycle du signal de synchronisation obtenu sur le second intervalle de temps ;

aligner temporellement le cycle respiratoire des premières données d'imagerie ultrasonore et des secondes données d'imagerie ultrasonore ; et

présenter une interface utilisateur graphique, GUI, (30) affichant une visualisation (34) incluant l'affichage simultané d'une image (36) de la première partie du système respiratoire du patient et d'une image (38) de la seconde partie du système respiratoire du patient généré respectivement à partir des premières données d'imagerie ultrasonore et des secondes données d'imagerie ultrasonore ;

dans lequel :

le second intervalle de temps est différent du premier intervalle de temps ;

l'affichage simultané de l'image de la première partie du système respiratoire du patient et de l'image de la seconde partie du système respiratoire du patient est généré respectivement à partir des premières données d'imagerie ultrasonore et des secondes données d'imagerie ultrasonore alignées temporellement ;

le cycle aligné du signal de synchronisation est utilisé pour aligner temporellement le cycle respiratoire des premières données d'imagerie ultrasonore et des secondes données d'imagerie ultrasonore ; et

**caractérisé en ce que** le signal de synchronisation est :

un signal de stimulation du nerf phrénique (SNP) indiquant une SNP administrée au patient sur les premier et second intervalles de temps ; ou

un signal d'épaisseur ou d'excursion du diaphragme extrait des premières données d'imagerie ultrasonore et des secondes données d'imagerie ultrasonore.

2. Dispositif de surveillance de la respiration selon la revendication 1, dans lequel :

soit le premier intervalle de temps est antérieur au second intervalle de temps, et l'alignement du cycle du signal de synchronisation, l'alignement temporel des premières données d'imagerie ultrasonore et des secondes données d'imagerie ultrasonore et l'affichage de la visualisation sont réalisés en temps réel pendant le second intervalle de temps ; et

soit l'alignement du cycle du signal de synchronisation, l'alignement temporel des premières données d'imagerie ultrasonore et des secondes données d'imagerie ultrasonore et l'affichage de la visualisation sont entièrement réalisés après l'achèvement à la fois du premier intervalle de temps et du second intervalle de temps.

3. Dispositif de surveillance de la respiration selon la revendication 1, dans lequel, dans la visualisation affichée :

l'image de la première partie du système respiratoire et l'image de la seconde partie du système respiratoire sont des images cinématographiques générées respectivement à partir des premières données d'imagerie ultrasonore et des secondes données d'imagerie ultrasonore ;

l'image cinématographique de la seconde partie du système respiratoire du patient est positionnée de manière controlatérale à l'image cinématographique de la première partie du système respiratoire du patient, en correspondance avec l'agencement controlatéral de la seconde partie du système respiratoire du patient par rapport à la première partie du système respiratoire du patient.

4. Dispositif de surveillance de la respiration selon la revendication 1, dans lequel le dispositif de commande électronique est en outre configuré pour :

déterminer une première valeur d'au moins un paramètre de diaphragme thoracique pour la première partie du système respiratoire du patient à partir des premières données d'imagerie ultrasonore ; et

déterminer une seconde valeur du au moins un paramètre de diaphragme thoracique pour la seconde partie du système respiratoire du patient à partir des secondes données d'imagerie ultrasonore ;

dans lequel la visualisation inclut en outre l'affichage de représentations des première et seconde valeurs du au moins un paramètre de diaphragme thoracique.

5. Dispositif de surveillance de la respiration selon la revendication 4, dans lequel le au moins un paramètre de diaphragme thoracique inclut au moins une (i) d'une mesure de l'épaisseur de diaphragme thoracique et/ou (ii) d'une mesure de l'excursion de diaphragme thoracique.

6. Dispositif de surveillance de la respiration selon la revendication 1, dans lequel le dispositif de commande électronique est configuré pour :
aligner positionnellement les premières données d'imagerie ultrasonore et les secondes données d'imagerie ultrasonore en alignant des images des parties du diaphragme thoracique représentées dans les premières données d'imagerie ultrasonore et les secondes données d'imagerie ultrasonore respectives.

7. Dispositif de surveillance de la respiration selon la revendication 6, dans lequel l'alignement positionnel inclut en outre la réalisation d'une déformation d'image pour supprimer une différence entre l'excursion du diaphragme sur le cycle respiratoire du système respiratoire du patient des parties du diaphragme thoracique représentées dans les premières données d'imagerie ultrasonore et les secondes données d'imagerie ultrasonore respectives.

8. Dispositif de surveillance de la respiration selon la revendication 1, dans lequel le dispositif de commande électronique est configuré pour :
effectuer une correction de mouvement pour supprimer au moins une composante de mouvement d'au moins une des premières données d'imagerie ultrasonore et des secondes données d'imagerie ultrasonore, dans lequel la au moins une composante de mouvement supprimées ne sont pas corrélées au cycle respiratoire des poumons du patient.

9. Dispositif de surveillance de la respiration selon la revendication 1, incluant en outre au moins un parmi :

un dispositif d'imagerie par ultrasons présentant une sonde d'imagerie par ultrasons pouvant être agencée par rapport au premier poumon et/ou à la première partie du diaphragme thoracique du patient pour acquérir les premières données d'imagerie ultrasonore et pouvant être agencée par rapport au second poumon et/ou à la seconde partie du diaphragme thoracique du patient pour acquérir les secondes données d'imagerie ultrasonore, dans lequel la sonde d'imagerie ultrasonore est commandée pour acquérir des données d'imagerie supplémentaires du diaphragme du patient sur la base des premières données d'imagerie ultrasonore et des secondes données d'imagerie ultrasonore alignées temporellement ;

un ventilateur mécanique configuré pour administrer une thérapie de ventilation mécanique au patient, dans lequel la réception des données d'imagerie d'une dimension des poumons se produit pendant l'inspiration et l'expiration, tandis que le patient suit une thérapie de ventilation mécanique ; dans lequel le ventilateur mécanique est commandé pour ajuster un ou plusieurs paramètres de la thérapie de ventilation mécanique administrée au patient sur la base des premières données d'imagerie ultrasonore et des secondes données d'imagerie ultrasonore alignées temporellement ; et

un dispositif de stimulation du nerf phrénique (PNS) configuré pour fournir un traitement de PNS au patient, dans lequel le dispositif de PNS est commandé pour ajuster un ou plusieurs paramètres de la thérapie de PNS délivrée au patient sur la base des premières données d'imagerie ultrasonore et des secondes données d'imagerie ultrasonore alignées temporellement.

10. Procédé de surveillance de la respiration (100) pour surveiller le système respiratoire d'un patient, le système respiratoire comprenant le poumon gauche, le poumon droit et le diaphragme thoracique, le procédé de surveillance de la respiration comprenant :

la réception de premières données d'imagerie ultrasonore (102) en fonction du temps acquises d'une première partie du système respiratoire du patient sur un premier intervalle de temps ;
la réception de secondes données d'imagerie ultrasonore (105) en fonction du temps acquises d'une seconde partie du système respiratoire du patient sur un second intervalle de temps dans lequel la seconde partie du système respiratoire du patient est controlatérale à la première partie du système respiratoire du patient ;
l'obtention d'un signal de synchronisation (103) pour le premier intervalle de temps et le second intervalle de temps dans lequel le signal de synchronisation est un cycle de signal unidimensionnel en correspondance avec le cycle respiratoire du système respiratoire du patient ;

l'alignement (107) du cycle du signal de synchronisation obtenu sur le premier intervalle de temps et du cycle du signal de synchronisation obtenu sur le second intervalle de temps ;

l'alignement temporel (108) du cycle respiratoire des premières données d'imagerie ultrasonore et des secondes données d'imagerie ultrasonore ; et

la présentation d'une interface utilisateur graphique (GUI) affichant (110) une visualisation incluant l'affichage simultané d'une image de la première partie du système respiratoire du patient et d'une image de la seconde partie du système respiratoire du patient généré respectivement à partir des premières données d'imagerie ultrasonore et des secondes données d'imagerie ultrasonore ;

dans lequel :

le second intervalle de temps est différent du premier intervalle de temps ;

l'affichage simultané de l'image de la première partie du système respiratoire du patient et de l'image de la seconde partie du système respiratoire du patient est généré respectivement à partir des premières données d'imagerie ultrasonore et des secondes données d'imagerie ultrasonore alignées temporellement ;

le cycle aligné du signal de synchronisation est utilisé pour aligner temporellement le cycle respiratoire des premières données d'imagerie ultrasonore et des secondes données d'imagerie ultrasonore ; et

**caractérisé en ce que** le signal de synchronisation est :

un signal de stimulation du nerf phrénique (SNP) indiquant une SNP administrée au patient sur les premier et second intervalles de temps ; ou

un signal d'épaisseur ou d'excursion de diaphragme extrait des premières données d'imagerie ultrasonore et des secondes données d'imagerie ultrasonore.

**Figure 1**

Mechanical
ventilation
method or process
100

FIG. 2

**100**

Position US probe to image
first portion of the
respiratory system <u>101</u>

first
time
Int.

Acquire first US imaging
data of first portion of the
respiratory system <u>102</u>

Synch signal
obtained for first
time interval <u>103</u>

Re-position US probe to
image second portion of
the respiratory system <u>104</u>

second
time
Int.

Acquire second US imaging
data of second portion of
the respiratory system <u>105</u>

Synch signal
obtained for second
time interval <u>106</u>

To Figure 3B

# Figure 3A

Align cycling of synch signal obtained
for the first and second time intervals
107

Temporal alignment of first ultrasound imaging
data and second ultrasound imaging data 108

Positional alignment of first ultrasound imaging
data and second ultrasound imaging data 109

Present GUI displaying
visualization including
simultaneous display of
contralateral images
respectively generated from
the aligned first ultrasound
imaging data and second
ultrasound imaging data 110

Control operation of
mechanical ventilator, PNS
device, or US imaging probe
111

# Figure 3B

Figure 4

Figure 5

34

32

Synchronization signal

Ventilator flow
Ventilator pressure
Ventilator volume
Work of breathing
Phrenic nerve stimulation
US diaphragm thickness
US diaphragm excursion

40

Ventilator
pressure

Live:
18:26:34

Time

36

38

Live: 18:26:34
Tfdi=_____

Playback: 18:19:56
Tfdi=_____

# Figure 6

# EP 4 622 557 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 63426931 B

- US 2019328361 A1

### Non-patent literature cited in the description

- **TUINMAN PR** ; **JONKMAN AH** ; **DRES M** ; **SHI ZH** ; **GOLIGHER EC** ; **GOFFI A** ; **DE KORTE C** ; **DEMOULE A** ; **HEUNKS L.** Respiratory muscle ultrasonography: methodology, basic and advanced principles and clinical applications in ICU and ED patients - a narrative review. *Intensive Care Med.*, 14 January 2020, vol. 46 (4), 594-605

- **MATAMIS DIMITRIOS et al.** Sonographic evaluation of the diaphragm in critically ill patients. *Technique and clinical applications*

- **RABBI, MOHAMMAD** ; **PIZZOLATO, CLAUDIO** ; **LLOYD, DAVID** ; **CARTY, CHRISTOPHER** ; **DEVA-PRAKASH, DANIEL** ; **DIAMOND, LAURA**. Non-negative matrix factorisation is the most appropriate method for extraction of muscle synergies in walking and running. *Nature Scientific Reports*, 2020